Europäisches Patentamt

European Patent Office

Office européen des brevets

(11) Publication number: **0 048 497**
**A2**

(12) # EUROPEAN PATENT APPLICATION

(21) Application number: **81107594.4**

(22) Date of filing: **23.09.81**

(51) Int. Cl.³: **C 12 N 15/00**
**C 12 N 1/20**
**//C12R1/19**

(30) Priority: **24.09.80 JP 131457/80**

(43) Date of publication of application:
**31.03.82 Bulletin 82/13**

(84) Designated Contracting States:
**DE FR GB**

(71) Applicant: **KYOWA HAKKO KOGYO CO., LTD.**
**Ohtemachi Bldg., 6-1 Ohtemachi Itchome**
**Chiyoda-ku Tokyo 100(JP)**

(72) Inventor: **Itoh, Seiga**
**218-14, Aihara Sagamihara-shi**
**Kanagawa-ken(JP)**

(74) Representative: **Vossius.Vossius.Tauchner.Heunemann.Rauh**
**Siebertstrasse 4 P.O. Box 86 07 67**
**D-8000 München 86(DE)**

(54) DNA transducing vector and microorganism containing it.

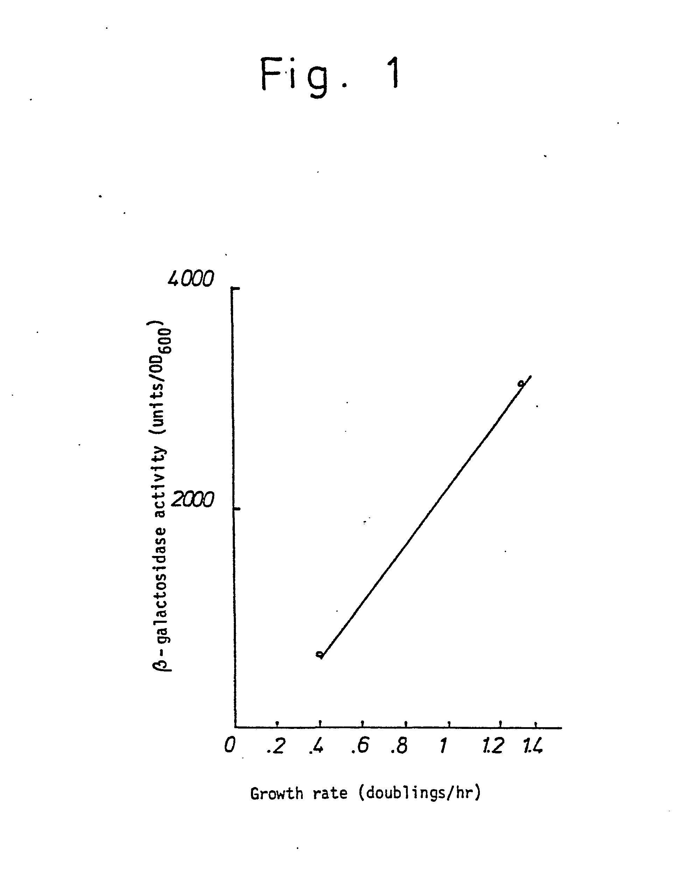

(57) Described is a DNA transducing vector carrying the $P_1$ promoter derived from ribosomal RNA of *Escherichia coli*, a microorganism containing a DNA transducing vector carrying this $P_1$ promoter, a recombinant DNA carrying this $P_1$ promoter, a microorganism containing this recombinant DNA, a microorganism containing said recombinant DNA and E. coli I-001 and I-002, ATCC No. 31 698 and 31 699. The $P_1$ promoter derived from ribosomal RNA of E. Coli has a stronger activity than the lactose promoter; see also *Fig. 1*.

Fig. 1

EP 0 048 497 A2

Croydon Printing Company Ltd.

0048497

"A DNA TRANSDUCING VECTOR CARRYING THE $P_1$ PROMOTER DERIVED FROM RIBOSOMAL RNA OF E. COLI, A MICROORGANISM CONTAINING A DNA TRANSDUCING VECTOR CARRYING THIS $P_1$ PROMOTER, A RECOMBINANT DNA CARRYING THIS $P_1$ PROMOTER, A MICROORGAN-ISM CONTAINING SAID RECOMBINANT DNA AND E. COLI I-001 AND I-002, ATCC No. 31 698 AND 31 699"

## TITLE MODIFIED
see front page

Background of the Invention

The present invention relates generally to a novel DNA transducing vector and a novel recombinant DNA, and more specifically to a DNA transducing vector carrying a $P_1$ promoter derived from ribosomal RNA of Escherichia coli and a recombinant DNA carrying the $P_1$ promoter.

DNA transducing vectors and recombinant DNAs are useful in recombinant technology for cloning genes of animals, plants and microorganisms and the expression thereof.

Recently the development of recombinant DNA technology has yielded a number of transducing vectors. One of the vectors which is most frequently used for the expression of the cloned gene for proteins derived from animals in Escherichia coli is a transducing vector carrying a lactose promoter.

Lactose operon, the operon for the lactose-utilizing system in Escherichia coli, is one of the well known operons. Three genes which encode β-galactosidase, galactoside permiase and galactoside acetyltransferase are aligned downstream of the lactose promoter and are expressed under the control of the promoter.

The available literature illustrates several examples wherein animal genes are cloned in transducing vectors carrying lactose promoters and the expression thereof has been ascertained. For example, Itakura et al. has succeeded in the expression of somatostatin (human hormone) gene which is synthesized by chemical methods and fused to a lactose promoter on the plasmid pBR 322 [K. Itakura et al., Science 198, 1056 (1978)]. Thereafter, chicken ovalbumin gene [B. J. Bruce, Proc. Natl. Acad. Sci., 75, 5936 (1978)], human insulin gene [A. D. Riggs et al., Proc. Natl. Acad. Sci., $\underline{76}$, 106 (1979)] and human growth hormone gene [D. V. Goeddel et al., Nature $\underline{281}$, 544 (1979)] have been fused to plasmids carrying a lactose promoter and the production of animal proteins in Escherichia coli has been confirmed. In the case of human growth hormone, 186,000 molecules of hormone per cell of E. coli were produced, which illustrates the usefulness of plasmid vectors having lactose promoters in recombinant technology.

The ribosome of E. coli is a complex (70S, M.W.: $2.8 \times 10^6$ dalton) of three RNAs (16S, 23S and 5S) and 55 proteins. The ribosome is an important intracellular particle which effects the synthesis of proteins and is composed of two particles, 30S and 50S. More precisely, one particle (30S) is a ribonucleoprotein composed of an RNA (16S, M.W.: $5.6 \times 10^5$ dalton) and 21 proteins and the other particle (50S) is that composed of two RNAs (23S, M.W.: $1.1 \times 10^6$ dalton, 5S, M.W.: $4 \times 10^4$ dalton) and 34 proteins.

In general, the process wherein a messenger RNA is synthesized using a DNA encoding genetic information as a template is called "transcription" and the process wherein a protein is synthesized depending on the information encoded on a messenger RNA is called "translation". Ribosomes play the most important role in the translation process.

It is said that about 15,000 ribosomes are generally present in one cell of E. coli but the amount thereof varies depending on the growth rate of the microorganism. That is, the amount of ribosome is proportional to the growth rate of the microorganism. It is known that the growth rate of E. coli in a nutrient-rich medium is high, i.e. about one division per 20 minutes and under such condition ribosomal RNAs increase in proportion to the growth rate of the microorganism [N. O. Kjeldgaard, J. Mol. Biol, $\underline{6}$, 341 (1963)].

Ribosomal RNA genes of E. coli are lined in the order of two promoters $P_1$ and $P_2$, 16S RNA, 23S RNA and 5S RNA. The synthesis of the three RNAs are under the control of the promoters $P_1$ and $P_2$. Such ribosomal RNA genes (operon) of E. coli are present in the ratio of 7 to one chromosome and are called ribosomal RNA gene (rrn) A, B, C, D, E, F and G, respectively [M. Nomura: Ann. Rev. Genet., 11, 297 - 347 (1977)]. It is known that as a feature of these RNA operons, tandem promoters named $P_1$ and $P_2$ are present, that is, $P_1$ and $P_2$ are located at about 300 base pairs and about 200 base pairs upstream from the 5' end of the structural gene 16S RNA, respectively.

It has been presumed, based upon in vitro experiments, that the intensity of the promoter $P_1$ (so called "transcription rate" on the synthesis of RNA from DNA) is 5 to 10 times that of $P_2$ [H. deBoer; J. Biol. Chem., 254, 5609 (1979)]. The results suggest that $P_1$ is more useful than $P_2$ in application to recombinant technology. The base sequences of the $P_1$ and $P_2$ promoters on the ribosomal RNA operon A, D and E have already been established and it has been determined that the $P_1$ promoter of these operons has the same 15 base pairs sequence including the so-called "Pribnow Box", a site to which RNA polymerase binds during transcription [H. deBoer et al., Cell, 17, 201 (1979), R. A. Young et al., Cell, 17, 225 (1979)].

Summary of the Invention

In accordance with the present invention, a DNA transducing vector and a recombinant DNA carrying the $P_1$ promoter has been made and it has been found that the $P_1$ promoter has a stronger promoter activity than that of the lactose promoter.

A comparison of the activities of these promoters shows that the $P_1$ promoter derived from ribosomal RNA of Escherichia coli (referred to as "$P_1$ fragment", hereinafter) has a stronger activity than the lactose promoter. Therefore, a DNA transducing vector or a recombinant DNA containing the $P_1$ promoter is useful for cloning genes of animals, plants and microorganisms and also the expression thereof.

## Detailed Description of the Invention

In accordance with the present invention, the following general procedures are employed.

(1)  Method for preparation of DNA transducing vector with $P_1$ fragment:

By way of example, the method of inserting the $P_1$ fragment into plasmid vector pBR 325 is explained.

Generally speaking, the $P_1$ fragment is prepared from the transducing phage DNA, λmet A 20.  Ribosomal RNA gene is cloned in the transducing phage DNA and ribosomal RNA promoter is included therein.

The phage DNA is digested with a restriction enzyme and the desired $P_1$ fragment is purified by agarose gel electrophoresis or polyacrylamide gel electrophoresis.  The $P_1$ fragment is inserted into a plasmid vector pBR 325.  The above processes are carried out according to the methods used generally in recombinant technology.  That is, the pBR 325 is digested with a restriction enzyme such as Eco RI, Hind III and Bam HI and the $P_1$ fragment is combined with the digested pBR 325 using T4DNA ligase.  As is illustrated in Flow Sheet I, the $P_1$ fragment has Eco RI and Hind III restriction sites at both ends.  When the $P_1$ fragment is inserted into Eco RI and Hind III sites of pBR 325 (refer to Flow Sheet II), the Hind II site of the $P_1$ fragment is changed to a Hind III site with the Hind III linker prior to the insertion.  The recombinant plasmid of $P_1$ fragment and pBR 325 is introduced into an E. coli strain and the transformant, grown on an agar medium containing 1 to 100 µg/ml, preferably about 20 µg/ml of ampicillin, is readily selected for tetracycline resistance and chloramphenicol sensitivity.

The construction of the recombinant plasmid is carried out under the following conditions. Digestion of DNA with restriction enzymes is carried out as follows. Generally 0.1 to 20 µg of DNA is digested with 0.1 to 100 units, preferably 1 to 3 units of a restriction enzyme per 1 µg of DNA in the presence of 2 to 200 mM, preferably 10 to 40 mM Tris-HCl (pH 6.0 to 9.5, preferably pH 7.0 to 8.0), 2 to 100 mM NaCl and 2 to 20 mM, preferably 5 to 10 mM $MgCl_2$ at a temperature of 18 to 42°C, preferably 32 to 38°C, for 15 minutes to 24 hours. The reaction is terminated by heating generally at 55 to 70°C, preferably 63 to 67°C for 5 to 10 minutes. Alternatively, the reaction may be terminated by inactivating the restriction enzyme by the addition of an agent such as diethylpyrocarbonate.

In the case of ligating two or more DNA fragments cleaved with restriction enzyme, the fragments are incubated with 0.3 to 10 units of T4 DNA ligase at a temperature of 1 to 37°C, preferably 3 to 20°C for 15 minutes to 72 hours, preferably 2 to 20 hours in the presence of 2 to 200 mM, preferably 10 to 40 mM Tris-HCl (pH 6.0 to 9.5, preferably pH 7.0 to 8.0), 2 to 20 mM, preferably 5 to 10 mM $MgCl_2$, 0.1 to 10 mM, preferably 0.5 to 2 mM ATP and 1 to 50 mM, preferably 3 to 20 mM dithiothreitol.

Thus, a recombinant plasmid of pBR 325 and $P_1$ fragment, i.e., DNA transducing vector is constructed.

It should be appreciated that the $P_1$ fragment may be inserted into pBR 325 after digestion with Eco RI and Bam HI or Eco RI and Sal I. Moreover, the $P_1$ fragment can be inserted after the digestion of pBR 325 with Pst I.

Although the plasmid vector pBR 325 has been described above, any plasmid vector may be employed in the present invention in which the $P_1$ promoter is inserted and shows promoter activity. Preferably, a plasmid vector having restriction sites digestable with at least one member selected from (1) EcoRI, (2) Hind III and (3) a combination of Eco RI or Hind III and Bam HI, Sal I or Pst I is used. As the most preferable plasmid vectors, pBR 325, pBR 322, pBR 327, pBR 328 and pGA 22 are examples. Vector pBR 322 is described in Gene $\underline{2}$, 95 (1977) (F. Bolivar) and pBR 327 and pBR 328 are described in Gene $\underline{9}$, 287 (1980) (X. Soberon et al). These plasmid vectors are digested with Eco RI or Hind III and the $P_1$ fragment is inserted into the digested vector to obtain a DNA transducing vector carrying the $P_1$ promoter of ribosomal RNA. The pGA 22 vector is described in J. Bacteriol. $\underline{140}$, 400 (1979) (G. An et al). A DNA vector which carries the $P_1$ fragment and ampicillin resistance gene or a DNA vector which carries the $P_1$ fragment, ampicillin resistance gene and kanamycin resistance gene is constructed by inserting the $P_1$ fragment into pGA 22 after digestion with Eco RI and Hind III.

(2) Construction of recombinant DNA

To obtain a recombinant DNA carrying the $P_1$ fragment, the $P_1$ fragment can be directly inserted into a plasmid or phage DNA which has a structural gene coding for growth hormone, insulin, interferon and the like. Furthermore, a DNA transducing vector carrying the $P_1$ fragment can be inserted into such plasmid or phage DNA.

Such recombinant DNA can be constructed by making a $P_1$ fragment, a plasmid vector or phage vector and a structural gene independently and combining them in one step or successive steps.

In the combining process for the construction of the recombinant DNA, T4DNA ligase is used.

One example of such recombinant DNA is a recombinant of the $P_1$ fragment and a phage vector containing a gene for β-galactosidase. The $P_1$ promoter derived from ribosomal RNA is effective for the expression of β-galactosidase and the promoter activity is more than two times stronger than that of a lactose promoter. A process for constructing such recombinant DNA is as follows.

A phage DNA, λ459 DNA which is a source of the vector and a phage DNA, λRS 205-7 DNA which is a source of the vector and the structural gene, and the $P_1$ fragment are used.

The λ459 DNA and λRS 205-7 DNA are digested with Eco RI, Hind III and Sal I. The restriction enzyme map is illustrated in Flow Sheet IV wherein the length of λphage DNA is illustrated as 100% (1% of λphage DNA corresponds to about 480 base pairs) and the black band means deletion of DNA.

As illustrated in Flow Sheet IV, the digestion of λ459 DNA with Eco RI gives two DNA fragments of which the fragment at the right hand is named "R-fragment" which corresponds to about 42% of the λDNA. The digestion of λRS 205-7 DNA with Hind III gives 7 DNA fragments of which the fragment at the left end is named "L-fragment" which corresponds to about 53% of the λDNA.

In λRS 205-7 of Flow Sheets I and IV, lac Z means a gene for β-galactosidase, Trp A means a gene for tryptophane synthetase A protein, Trp B means a gene for tryptophane synthetase B protein, lac $Z^+$ and Trp $A^+$ mean the presence of the complete structural genes thereof, and Trp $B^-$ means the presence of a part of the structural gene thereof.

The base sequence which relates to the termination of transcription and exists between Trp A and lac Z, that is, terminator, and the base sequence which relates to the initiation of transcription of lac Z, that is, promoter, are eliminated by a genetic process. The eliminated region is named "Trp - Lac fusion" and W 205 (see Flow Sheet IV) is one of the Trp - Lac fusions [Mitchell et al., J. Mol Biol., 93, 331 (1975)].

The L-fragment carrying Lac Z and Trp A, which is obtained by the digestion of λRS 205-7 DNA with Hind III, the R-fragment obtained by the digestion of λ459 DNA with Eco RI and the $P_1$ fragment prepared as above are combined with T4 DNA ligase to make a recombinant DNA of "L-fragment (Lac $Z^+$, Trp $A^+$)" - $P_1$ fragment - "R-fragment". A strain of E. coli is transformed with the recombinant DNA and the transformant is used for the production of β-galactosidase.

As shown in the following examples, illustrating specific embodiments of the present invention, the amount of β-galactosidase produced by the transformant is two or more times that of an E. coli strain containing the lactose promoter. This fact illustrates the superiority of the $P_1$ fragment as a promoter.

Example 1

In this example a DNA transducing vector carrying the $P_1$ fragment is constructed as follows:

1)    Preparation of $P_1$ fragment

A transducing phage DNA carrying the $P_1$ promoter of ribosomal RNA gene-E, λmet A 20 DNA is prepared according to the method described in G. Zubay et al., The Lactose Operon, J. R. Beckwith eds (New York: Cold Spring Harbor Laboratory) p. 375 - 391; Yamamoto et al; FEBS Letters, 72, 256 (1976); Flow Sheet I. The λmet A 20 DNA is digested with a restriction enzyme Eco RI (product of Bethesda Research Laboratories Co., (hereinafter referred to as "B.R.L.") and the digest is subjected to purification by agarose gel electrophoresis to obtain a DNA fragment with 1,370 base pairs. The DNA fragment is digested with a restriction enzyme Hind II (product of B.R.L.) and a restriction enzyme Hind III (product of B.R.L.) and the digest is again subjected to purification by agarose gel electrophoresis to obtain a $P_1$ fragment with 500 base pairs.

Flow Sheet I shows a restriction map around the $P_1$ and $P_2$ promoters of ribosomal RNA on the phage λmet A 20 DNA and the size of each fragment.

λmet A 20 (rrnE)

2Kb

Sal I  12.0|2.0|12.5          ||15.0
Hin III  11.3||19          |5.0          ||          6.3|4.2

| purD | 16.5 | 23S | metA |

Sma I  11.6||13          7.6|7.8
Eco RI  10.3|1.3|2.0|12.2          7.2|5.7          |3.2

100 bp

EcoRI

Hind II          Hind III          EcoRI

500          300          570

P₁ → P₂ →          16s - rRNA

Hind III

0048497

2)    Preparation of $P_1$ fragment having a Hind III end:

   (1)  Materials

      The 5' end of Hind III linker [d (CCAAGCTTGG), product of Collaborative Research Inc.] is phosphorylated with ATP and polynucleotide kinase according to a conventional method of phosphorylation [Nucleic Acid Res., 5, 4479 (1978)] to obtain phosphorylated Hind III linker.  T4 DNA ligase is a product of New England Biolabo Co.

   (2)  Method

      1 µg of phosphorylated Hind III linker and 2 µg of the $P_1$ fragment obtained as above are combined at both blunt ends by incubating with 1 unit of T4 DNA ligase at 4°C for 20 hours in the presence of 6.6 mM Tris-HCl (pH 7.6), 6.6 mM $MgCl_2$, 1 mM ATP and 10 mM dithiothreitol.  The total volume of the reaction solution is 30 µl.

      The reaction is terminated by heating at 65°C for 5 minutes and the reaction solution is digested with 4 units of Hind III and 4 units of Eco RI at 37°C for 4 hours.  All of the reaction mixture (40 µl) is charged on a column packed with 2 ml of Sephadex G-100 and elution is carried out with a mixture of 20 mM Tris-HCl (pH 7.8), 50 mM NaCl and 10 mM disodium ethylenediaminetetraacetate (EDTA) (concentrations given are the final) to remove extra Hind III linker.

      Then, 0.6 ml (I.1 ml to 1.7 ml) of fractions are combined and 1.5 ml of ethanol is added.  The DNA precipitated is dried and suspended again in 20 mM Tris-HCl (pH 7.6) to make up 20 µl of a solution.

3)    Construction of recombinant plasmid:

   (1)  Materials

      The plasmid vector pBR 325 [F. Bolivar,   Gene 4, 121 (1978)] prepared according to the method described in P. Guerry et al., J. Bacteriol. 116, 1064 (1973) is used.

(2) Method

With reference to the following Flow Sheet II, 5 µg of pBR 325 is digested with 10 units of Hind III and 10 units of Eco RI at 37°C for 2 hours in the presence of 20 mM Tris-HCl (pH 7.6), 60 mM NaCl and 7 mM $MgCl_2$ and the reaction is terminated by heating at 65°C for 5 minutes.

Then, 0.1 µg of the pBR 325 digested with Hind III and Eco RI and 5 µl of a solution containing 0.5 µg of the $P_1$ fragment having the Hind III end obtained as above are mixed and 10 µl of a solution of 20 mM Tris-HCl (pH 7.6), 10 mM $MgCl_2$, 10 mM dithiothreitol and 2 mM ATP are added to make up 20 µl of a solution.

Then, 0.5 unit of T4 DNA ligase is added to the reaction mixture and the mixture is kept at 4°C for 18 hours to make a recombinant plasmid of the $P_1$ fragment and pBR 325. The recombinant plasmid is named pKS 1003.

## F L O W   S H E E T   II

EcoRI

(base
500 pairs)

$P_1$ →

$P_1$-fragment   Hind III
0.5 μg

Hind III

pBR325   5 μg
($Ap^R$, $Tc^R$, $Cm^R$)

Digestion with
EcoRI + Hind III

65°C , 5 min
Reaction solution
(0.1 μg)

Ligation

Transformation

Select $Ap^R$, $Tc^R$, $Cm^S$ colonies

EcoRI

Hind III

$P_1$

pKS 1003

BamHI

Sal I

4.9 kilo base,   $Ap^R$, $Tc^R$, $Cm^S$

Example 2

In this example, a microorganism is prepared wherein a recombinant plasmid carrying the $P_1$ fragment is introduced.

The recombinant plasmid carrying the $P_1$ fragment obtained in Example 1, i.e. DNA transducing vector is introduced into a microorganism in the following manner.

Escherichia coli C 600 SF 8 [Cameron et al, Proc. Natl. Acad. Sci., 72, 3416 (1975)] is used as a recipient strain and transformation is performed according to S. N. Cohen's method [Proc. Natl. Acad. Sci., 69, 2110 (1972)].

Among the colonies grown on a bouillon agar plate containing 50 µg/ml ampicillin, transformants of E. coli C 600 SF 8 which carry plasmid pKS 1003 are obtained by selecting a tetracycline resistant ($TC^R$) and a chloramphenicol sensitive ($CM^S$) strain, because the parent plasmid pBR 325 carries genes for $AP^R$, $TC^R$ and $CM^R$ and the recombinant plasmid carrying the $P_1$ promoter derived from ribosomal RNA carries genes for $AP^R$, $TC^R$ and $CM^S$.

DNAs are prepared from E. coli C 600 SF 8 ($AP^R$, $TC^R$, $CM^S$) by a conventional method and digested with Eco RI and Hind III. The presence of the $P_1$ fragment (500 base pairs) is confirmed by agarose gel electrophoresis.

Insofar as the E. coli strain with the recombinant plasmid is $TC^R$ and the $P_1$ fragment is generated by the digestion with Eco RI and Hind III , the $P_1$ fragment is inserted in the clockwise direction as shown in Flow Sheet II.

The microorganism strain containing the DNA transducing vector obtained in this Example is named Escherichia coli I-001. The strain has been deposited with the American Type Culture Collection, U.S.A. and has been accorded accession number ATCC 31698.

BAD ORIGINAL

Example 3

In this example, a recombinant DNA carrying the $P_1$ fragment is constructed as follows with reference to the following Flow Sheet III illustrating the processes for constructing the recombinant DNA, and the following Flow Sheet IV illustrating the restriction maps of λ459 DNA, λRS 205-7 DNA and the recombinant DNA carrying the $P_1$ fragment.

First, 2 µg of the phage λRS 205-7 DNA digested with Hind III, 5 µg of the $P_1$ fragment having Hind III site and Eco RI site at both ends, and 2 µg of λ459 DNA digested with Eco RI are mixed in 40 µl of a solution containing 50 mM Tris-HCl (pH 7.6), 5 mM $MgCl_2$, 5 mM dithiothreitol and 1 mM ATP. Then, 1 unit of T4 DNA ligase is added and the mixture is allowed to react at 4°C for 20 hours.

As a result, a recombinant DNA wherein the L-fragment of λRS205-7 DNA, $P_1$ fragment and R-fragment of λ459 DNA combined in this order is obtained. The recombinant DNA carries Lac Z and Trp A on the L-fragment. Detection of the Lac Z gene (β-galactosidase gene) on the recombinant DNA is performed by subjecting the recombinant DNA to transfection using E. coli $LacO^-Z^-$, a mutant strain which has no operator and structural gene for β-galactosidase, as an indicator strain. The phage containing the recombinant DNA is detected as a blue plaque on a bouillon agar plate containing 5-bromo-4-chloro-3-indoyl-β-D-galactoside.

The thus obtained phage containing the recombinant DNA is named λ$P_1$-Lac 1.

The presence of the $P_1$ promoter on λ$P_1$-Lac 1 DNA is confirmed by the fact that the $P_1$ fragment (500 base pairs) is generated when λ$P_1$-Lac 1 DNA is digested with Hind III and Eco RI.

# F L O W   S H E E T   III

## (Phage cloning system)

λ RS-205 ( L )    III-L  P₁    λ459 ( R )

| LacZ | | III | P₁ | | R1 | | |

2 μg        5 μg        .2 μg

Ligation

Transfection (KH802 r⁻m⁺)

X-Gal-LB(5-Br-4Cl-3-Indoyle-β-D-
Galactoside)

LacOZ⁻ Strain (Indicator)

Blue plaque

Lysate (30 ml)

CsCl (x2)

Phenol Extraction

DNA

III: Hind III
III-L: Hind III Linker

F L O W   S H E E T   IV

- 91 -

Example 4

In this example, a microorganism strain is prepared wherein a recombinant DNA is introduced.

An Escherichia coli strain wherein the gene for β-galactosidase is deleted, i.e. Escherichia coli B 2550 Lac O⁻Z⁻Y^CTrp⁻ [Y^C means constitutive property for permease and Trp⁻ means requirement for tryptophan] is lysogenized with the recombinant phage obtained in Example 3. As a control, a strain lysogenized with λ plac 5 which is a phage carrying the lactose operon of E. coli [K. Ippen et al., J. Bact., 108, 5 - 9 (1971)] is used.

The lysogenic strains are inoculated into 2 ml of LB broth wherein 10 g of bactotryptone, 5 g of yeast extract and 5 g of NaCl are dissolved in 1 ℓ of water and the solution is adjusted to pH 7.0 with NaOH and 2 ml of LB broth containing 1 mM isopropylthiogalactoside (IPTG) (referred to as "LB + IPTG broth" hereinafter), and cultivated at 30°C for 18 hours. Then, 0.25 ml of the cultures are inoculated into 5 ml of the same brothes and the cultivation is carried out at 30°C for 3 hours. When the absorbance at 600 nm ($OD_{600}$) reaches 0.3 to 0.6, β-galactosidase activity of each culture is determined by Miller's method [J. H. Miller: Experiments in Molecular Genetics, p. 352 (1972), Cold Spring Harbor Laboratory]. The results are shown in Table 1.

Table 1

| Microorganism | β-galactosidase (unit/$OD_{600}$) | |
| --- | --- | --- |
| | L B broth | LB + IPTG broth |
| Parent strain (B2550) | 4 | 6 |
| B2550 (λ plac 5) | 35 | 1,532 |
| B2550 (λ $P_1$-Lac 1) | 4,204 | 4,260 |

The results show that the $P_1$ fragment acts as a promoter in the recombinant DNA. Moreover, the amount of β-galactosidase produced by B2550 ($\lambda P_1$-Lac 1) is at least two fold more than that of B2550 ($\lambda$plac 5), thus illustrating the improved promoter ability of the $P_1$ fragment over the known promoter.

The lysogenic strain with $\lambda P_1$-Lac 1 is named <u>Escherichia</u> <u>coli</u> I-002. The strain has been deposited with the American Type Culture Collection, U.S.A. and accorded accession number ATCC 31699.

## Example 5

In this example, the production of β-galactosidase using recombinant DNA is examined.

The lysogenic strain with the recombinant DNA ($\lambda P_1$-Lac 1) obtained in Example 4, <u>Escherichia</u> <u>coli</u> I-002, is cultured in the following three media to examine the correlation between growth rate and productivity of β-galactosidase.

A medium : 0.4% Glucose is added to a minimum medium which consists of 6 g/l $Na_2HPO_4$, 3 g/l $KH_2PO_4$, 0.5 g/l NaCl, 1 g/l $NH_4Cl$ and 0.1 mM $CaCl_2$ (the same as hereinafter).

B medium : A minimum medium containing 0.4% glucose and 0.4% casamino acid.

C medium : An LB broth containing 0.4% glucose.

Culturing is carried out at 30°C.

The results are shown in Fig. 1. The results show the production of β-galactosidase by the lysogenic strain with $\lambda P_1$-Lac 1 increases greatly in a medium wherein the strain grows rapidly which coincides with the information that ribosomal RNA increases in proportion to the growth of the strain [N. O. Kjeldgaard: J. Mol. Biol, <u>6</u>, 341 (1963)].

WHAT IS CLAIMED IS:

1.  A DNA transducing vector carrying the $P_1$ promoter derived from ribosomal RNA of Escherichia coli.

2.  A microorganism containing a DNA transducing vector carrying the $P_1$ promoter derived from ribosomal RNA of Escherichia coli.

3.  Escherichia coli I-001, ATCC 31698.

4.  A recombinant DNA carrying the $P_1$ promoter derived from ribosomal RNA of Escherichia coli.

5.  A microorganism containing a recombinant DNA carrying the $P_1$ promoter derived from ribosomal RNA of Escherichia coli.

6.  Escherichia coli I-002, ATCC 31699.

# Fig. 1

Growth rate (doublings/hr)